# EUROPÄISCHE PATENTSCHRIFT

(11) **EP 3 204 061 B1**
(45) Veröffentlichungstag und Bekanntmachung des Hinweises auf die Patenterteilung: **19.08.2020**
(21) Anmeldenummer: 15790470.7
(22) Anmeldetag: 09.10.2015
(51) Int. Cl.: A61L 31/10, A61L 31/16

(54) **GEFÄSSENDOPROTHESENBESCHICHTUNG**
COATING OF A VASCULAR ENDOPROSTHESIS
REVÊTEMENT D'ENDOPROTHÈSE VASCULAIRE

(30) Priorität: 10.10.2014 DE 102014014771
(43) Veröffentlichungstag der Anmeldung: 16.08.2017
(73) Patentinhaber: Aachen Scientific International PTE. LTD., 079903 Singapore (SG)
(72) Erfinder: RÜBBEN, Alexander, 98000 Monaco (MC)
(74) Vertreter: Schneiders, Josef
(86) Internationale Anmeldenummer: PCT/EP2015/073389
(87) Internationale Veröffentlichungsnummer: WO 2016/055612

(56) Entgegenhaltungen:
- WO-A1-2009/124570
- WO-A1-2013/178820

## Beschreibung

Die Erfindung betrifft ein Verfahren zur Beschichtung einer Gefäßendoprothese. Darüber hinaus betrifft die Erfindung die durch das Verfahren erhältliche Gefäßendoprothese.

Die sogenannten "minimalinvasiven Verfahren" nehmen in der Medizin einen immer größeren Stellenwert ein. So werden bei der Behandlung von Gefäßverengungen (Stenosen) häufig Gefäßendoprothesen, sog. Stents, in das Gefäß eingesetzt, um das Gefäß offen zu halten. Die Gefäßendoprothese weist typischerweise eine Röhrenform auf und besteht aus einer Geflecht- oder Gitterstruktur aus Metall oder Kunststoff. Sie kann eine komprimierte Form einnehmen, um sie durch einen Katheter an den Zielort einführen zu können. Am Zielort wird die Gefäßendoprothese dann aufgeweitet, so dass sie ihre expandierte Form einnimmt. Das Aufweiten kann mit Hilfe eines Ballons erfolgen. Bekannt sind auch selbstexpandierende Gefäßendoprothesen, die aus einem Formgedächtnismaterial bestehen und sich von selbst entfalten, sobald sie nicht mehr in ihrer komprimierten Form gehalten oder einer Temperaturänderung unterworfen werden. Das Verfahren der Erweiterung von verschlossenen oder verengten Blutgefäßen mit Hilfe einer Gefäßendoprothese wird auch als Stentangioplastie bezeichnet.

Als problematisch hat sich erwiesen, dass es bei Verwendung herkömmlicher Gefäßendoprothesen häufig nach einer gewissen Zeit durch Zellproliferation und Gewebeneubildung zu Restenosen kommt, d. h. das Gefäßlumen verengt sich erneut. Dem wird versucht, durch medikamentenbeschichtete Gefäßendoprothesen vorzubeugen (sog. drug eluting stents). Insbesondere kann es sich bei den Medikamenten um Proliferationshemmer wie Paclitaxel oder auch um Immunsuppressiva wie Sirolimus handeln. Die Beschichtung kann dadurch erzeugt werden, dass der Wirkstoff in einem Lösungsmittel gelöst auf die Gefäßendoprothese aufgebracht wird. Der Wirkstoff setzt sich auf diese Weise auf der Gefäßendoprothese ab und wird im Anschluss an die Implantierung nach und nach freigesetzt.

Typischerweise werden Beschichtungen auf der Gefäßendoprothese dadurch erzeugt, dass die Gefäßendoprothese mit einer Lösung des Wirkstoffs in Kontakt gebracht wird, beispielsweise in sie eingetaucht wird. Das Lösungsmittel wird anschließend entfernt, z. B. durch Verdunstung. Ebenso möglich ist die Ausfällung des Wirkstoffs durch Eintauchen der Gefäßendoprothese in eine Flüssigkeit, die sich zwar mit dem Lösungsmittel der Wirkstofflösung vermischt, in der der Wirkstoff selbst aber weitgehend unlöslich ist. Ein solches Verfahren wird in der WO 2009/124570 A1 beschrieben. Diese offenbart ein Verfahren zur Erzeugung einer bioaktiven Oberfläche auf einer Endoprothese, bei dem die Oberfläche der Endoprothese mit einer Lösung eines Wirkstoffs in einem mit Wasser mischbaren Lösungsmittel benetzt wird und die benetzte Endoprothese in Wasser getaucht wird, um den Wirkstoff auszufällen. Bei den vorgenannten Verfahren ergibt sich in der Regel eine kristalline, lackartige Beschichtung der Gefäßendoprothese. Es hat sich allerdings herausgestellt, dass die Wirkstoffabgabe von einer solchen Gefäßendoprothese im implantierten Zustand an die umgebende Gefäßwand nicht immer optimal ist.

Ausgehend von diesem Stand der Technik stellt sich die Aufgabe, ein Verfahren zur Herstellung einer Gefäßendoprothese bereitzustellen, das für eine verbesserte Abgabe des Wirkstoffs sorgt.

Diese Aufgabe wird erfindungsgemäß gelöst durch ein Verfahren zur Beschichtung einer Gefäßendoprothese mit folgenden Schritten:
a1) zumindest teilweise Benetzung der Gefäßendoprothese mit einer ersten Lösung eines hydrophoben Wirkstoffs
b) zumindest teilweise Benetzung der mit der ersten Lösung des Wirkstoffs benetzten Bereiche der Gefäßendoprothese mit einer Wasser und/oder mindestens einen Alkohol enthaltenden Flüssigkeit,
wobei vor der Benetzung der Gefäßendoprothese mit der Wasser und/oder mindestens einen Alkohol enthaltenden Flüssigkeit eine Trocknung der Gefäßendoprothese und eine Auskristallisation des Wirkstoffs erfolgt und wobei die Wasser und/oder mindestens einen Alkohol enthaltende Flüssigkeit einen Alkohol und/oder ein Keton enthält.

Es hat sich herausgestellt, dass die Benetzung mit der Wasser und/oder mindestens einen Alkohol enthaltenden Flüssigkeit die Wirkstoffoberfläche partiell angreift und diese poröser macht. Die Beschichtung wird somit spröder, optisch weniger transparent und milchiger. Wegen der kreideartigen Konsistenz der Oberfläche wird ein höherer Wirkstoffabtrag und eine höhere Wirkstoffabgabe an umgebende Gefäße erreicht, sobald die Gefäßendoprothese eingesetzt wurde, als es ohne die Benetzung mit der zusätzlichen, Wasser und/oder mindestens einen Alkohol enthaltenden Flüssigkeit der Fall wäre. Es wird vermutet, dass der Wasser- bzw. Alkoholanteil eine Änderung der Kristallstruktur des Wirkstoffs hervorruft.

Der Schritt der Benetzung mit der Wasser/Alkohol enthaltenden Flüssigkeit wird durchgeführt, nachdem die Gefäßendoprothese vollständig oder weitgehend getrocknet und der Wirkstoff auskristallisiert ist. Die Trocknung kann durch Bewegung der Gefäßendoprothese und/oder einen Luft- oder Gasstrom unterstützt werden. Ausgangspunkt für die zusätzliche Benetzung ist somit eine lackartige und transparente Wirkstoffschicht, die eine homogene und reproduzierbare Wirkstoffbeladung gewährleistet. Die Benetzung mit der Wasser/Alkohol enthaltenden Flüssigkeit erfolgt in den meisten Fällen durch Eintauchen, denkbar sind aber auch andere Formen der Benetzung, etwa durch Besprühen, Auftropfen o.ä..

Bei der Wasser und/oder mindestens einen Alkohol enthaltenden Flüssigkeit handelt es sich um eine einen Alkohol und/oder ein Keton enthaltende wässrige Lösung. Die Konzentration des Alkohols und/oder Ketons in der wässrigen Lösung beträgt typischerweise 10 bis 70 % (v/v), bevorzugt 20 bis 40 % (v/v) und besonders bevorzugt ca. 30 % (v/v). Verwendbar sind grundsätzlich mit Wasser mischbare Alkohole und Ketone, wobei auch eine Mischung aus mehreren Alkoholen und/oder Ketonen verwendet werden kann, für die dann die oben genannten, bevorzugten Konzentrationsangaben insgesamt gelten. Bevorzugt ist die Verwendung von Ethanol, Methanol, Aceton und/oder Isopropanol. Am meisten bevorzugt ist Ethanol. Weiterhin kann die wässrige Lösung ein azeotropes Lösungsmittelgemisch umfassen, insbesondere ein Alkohol/Wasser-Gemisch, bevorzugt ein Ethanol/WasserGemisch. Möglich ist auch der Zusatz einer kleinen Menge, typischerweise ca. 0,1 % (v/v) Essigsäure, wodurch eine Stabilisierung des Wirkstoffs, insbesondere von Paclitaxel, erreicht wird. Nach der Benetzung lässt man die Gefäßendoprothese trocknen, bevorzugt über einen Zeitraum ≥ 2 min, weiter bevorzugt ≥ 3 min, besonders bevorzugt ≥ 5 min. Während dieser Trocknung wird die Gefäßendoprothese bevorzugt rotiert und/oder einem Luft- oder Gasstrom ausgesetzt, um die Trocknung zu verbessern und überschüssiges Lösungsmittel zu entfernen. Eine Rotation kann auch bereits während der Benetzung der Gefäßendoprothese erfolgen. Denkbar ist auch, dass die Flüssigkeit eine zusätzliche Menge Wirkstoff enthält, um die Beladung der Gefäßendoprothese weiter zu erhöhen.

Da typische Gefäßendoprothesen eine Geflecht- oder Gitterstruktur aufweisen, der Umfang der Gefäßendoprothese somit eine Vielzahl von Durchlässen aufweist, erfolgt die Benetzung der Gefäßendoprothese mit dem Lösungsmittel, in dem der Wirkstoff gelöst ist, in der Regel sowohl innen als auch außen. Dies ist jedoch insofern unerwünscht, als der Wirkstoff auf der Innenseite der Gefäßendoprothese auch die Einbettung der Gefäßendoprothese in das körpereigene Gewebe behindert. Gemäß einer bevorzugten Ausführungsform wird das Verfahren daher so modifiziert, dass der Innenraum der Gefäßendoprothese weitgehend wirkstofffrei bleibt. Dies gelingt dadurch, dass man die Gefäßendoprothese in einem Schritt a2) in eine schnelle Rotationsbewegung um ihre Längsachse versetzt. Der Schritt a2) wird zwischen den Schritten a1) und b) durchgeführt. Die auftretende Zentrifugalkraft bewirkt, dass das Lösungsmittel, in dem der Wirkstoff gelöst ist, nach außen geschleudert wird und die Innenfläche bzw. der Innenbereich der Gefäßendoprothese praktisch wirkstofffrei bleibt. Die Gefäßendoprothese kann sich bereits während des Schritts a1), d.h. bei der Benetzung mit der Wirkstofflösung, in Rotation befinden. Dies gilt insbesondere, wenn die Benetzung durch Eintauchen in die entsprechende Lösung erfolgt. Alternativ kann die Gefäßendoprothese auch erst nach erfolgtem Schritt a1) in Rotation versetzt werden.

Die Umdrehungsgeschwindigkeit sollte mindestens 1.000 U/min, bevorzugt mind. 2.000 U/min und besonders bevorzugt mind. 5.000 U/min betragen. Als besonders vorteilhaft hat sich eine Umdrehungsgeschwindigkeit von 5.000 bis 10.000 U/min herausgestellt. Eine entsprechend hohe Rotationsgeschwindigkeit sorgt in effektiver Weise dafür, dass das den Wirkstoff enthaltende Lösungsmittel abgeschleudert wird und der innere Bereich der Gefäßendoprothese praktisch wirkstofffrei bleibt. Darüber hinaus werden auch Wirkstoffreste entfernt, die die Zwischenräume der Gefäßendoprothese ganz oder teilweise überspannen oder in diese hineinragen. Derartige Wirkstoffreste sind unerwünscht, da es sich nicht um eine reproduzier- und quantifizierbare Wirkstoffmenge handelt. Man lässt die Gefäßendoprothese typischerweise über einen Zeitraum von 10 s bis 2 min nach der Benetzung mit der Wirkstofflösung rotieren, ein Zeitraum von 30 s hat sich als in der Regel ausreichend herausgestellt. Die Rotationsgeschwindigkeit ist erheblich höher als bei teilweise aus dem Stand der Technik bekannten Verfahren, bei denen durch eine Bewegung eine gleichmäßige Verteilung des Wirkstoffs oder ein Trocknen erreicht werden soll.

Da das Innere der Gefäßendoprothese weitgehend wirkstofffrei bleibt, wird die Gefäßendoprothese schneller mit Endothel überzogen. Im gleichen Maße verringert sich die Anregung der Blutgerinnung durch die Gefäßendoprothese. Durch Verwendung der erfindungsgemäßen Gefäßendoprothesen ist es daher möglich, schneller auf Blutgerinnungshemmer wie Acetylsalicylsäure zu verzichten. Gleichzeitig werden aber Restenosen durch die Aufbringung des Wirkstoffs auf der Außenseite der Gefäßendoprothese wirkungsvoll verhindert.

Die Kristallisation des Wirkstoffs auf Gefäßendoprothesen hat sich zum Teil als schwierig herausgestellt. Hintergrund ist, dass, anders als etwa bei den Ballons für die Ballonangioplastie, die Oberfläche der Gefäßendoprothese hydrophil und nicht hydrophob ist, weshalb sich auf der Oberfläche der Gefäßendoprothese nicht ohne Weiteres Kristallisationskeime der hydrophoben Wirkstoffe bilden.

Gemäß einer besonders bevorzugten Ausführungsform wird daher in einem Schritt a3) eine radial wirkende mechanische Kraft auf die Außenseite der Gefäßendoprothese ausgeübt. Auf diese Weise wird erreicht, dass der Wirkstoff auf der äußeren Oberfläche gut kristallisiert. Der Schritt a3) erfolgt zwischen den Schritten a1) und b) bzw., wenn auch der Schritt a2) ausgeführt wird, zwischen den Schritten a2) und b).

Im Schritt a3) wird durch Ausübung einer radial wirkenden mechanischen Kraft auf die Außenseite der Gefäßendoprothese erreicht, dass sich Kristallisationskeime für den Wirkstoff bilden, so dass der hydrophobe Wirkstoff auf der hydrophilen Oberfläche der Gefäßendoprothese gut kristallisiert. Für die radial wirkende Kraft ist von Bedeutung, dass sie gleichmäßig über den gesamten Umfang auf der Außenseite der Gefäßendoprothese in den Bereichen einwirkt, in denen eine Benetzung stattgefunden hat. Unter radial wird eine Kraft verstanden, die von außen über den Umfang einwirkt, im Gegensatz zu einer axial wirkenden Kraft auf die Längsenden der Gefäßendoprothese.

Insbesondere kann eine radial einwirkende mechanische Kraft dadurch ausgeübt werden, dass die Gefäßendoprothese über eine Oberfläche gerollt wird. Dabei kann es sich um eine Elastomeroberfläche, beispielsweise eine Gummioberfläche handeln. Der auf die Gefäßendoprothese ausgeübte Druck sollte konstant bleiben, so dass die Kraftbeaufschlagung der Gefäßendoprothese über den gesamten Umfang gleichmäßig ist. Beim Rollen der Gefäßendoprothese über die Oberfläche werden Kristallisationskeime erzeugt, die für die Ausbildung einer kristallisierten Wirkstoffbeschichtung von Bedeutung sind. Um beim Rollen über eine Oberfläche keine Deformierung der Gefäßendoprothese zu verursachen, ist es sinnvoll, den Innenraum der Gefäßendoprothese auszufüllen, beispielsweise mit einem in Längsrichtung eingeschobenen Stab, der vorzugsweise aus Glas oder Metall bestehen kann.

Selbstverständlich sind auch alternative Formen der Ausübung einer radial einwirkenden mechanischen Kraft denkbar. Beispielsweise kann die Gefäßendoprothese in ein entsprechendes Werkzeug eingeführt werden, das für die Ausübung einer gleichmäßigen, radial einwirkenden mechanischen Kraft geeignet ist. Die Kraft muss so bemessen sein, dass eine unerwünschte Verformung der Gefäßendoprothese nicht stattfindet.

Unabhängig von der Art der Kraftausübung, durch Rollen über eine Oberfläche oder auf andere Weise, ist typischerweise ein leichtes Andrücken ausreichend, beispielsweise mit einer Kraft von 0,5 bis 5 N, vorzugsweise 1 bis 3 N, z. B. 2 N.

Die erste Lösung kann hinsichtlich des Wirkstoffs gesättigt sein. Als Lösungsmittel können beispielsweise Dichlormethan, Chloroform, ein Alkohol, insbesondere Ethanol, Methanol oder Isopropanol, Aceton, Diethylether, flüssige Kohlenwasserstoffe, wie zum Beispiel Pentan, Hexan, Heptan, Cyclohexan oder Octan, Toluol, Tetrahydrofuran (THF), Dimethylsulfoxid (DMSO), Dioxan, Dimethylformamid (DMF) oder Essigester verwendet werden. Möglich ist auch die Verwendung von Lösungsmittelgemischen. Vorzugsweise handelt es sich um eine Lösung des Wirkstoffs in Chloroform oder Dichlormethan, wobei Chloroform insofern bevorzugt ist, als es sich langsamer verflüchtigt und daher mehr Zeit verbleibt, innerhalb der das Lösungsmittel zusammen mit dem Wirkstoff durch die Rotationsbewegung nach außen geschleudert werden kann.

Eine typische Konzentration für den Wirkstoff in der ersten Lösung liegt in einem Bereich von 50 - 500 mg/ml, insbesondere 100 bis 300 mg/ml. Diese Konzentrationen haben sich bei Ausbildung einer Paclitaxel-Beschichtung bewährt. Grundsätzlich kann es sich auch um eine gesättigte Wirkstofflösung handeln.

Das Herausbewegen der Gefäßendoprothese aus der ersten Lösung nach erfolgter Benetzung mit Wirkstofflösung erfolgt bevorzugt mit einer Geschwindigkeit von ≤ 50 mm/s, weiter bevorzugt ≤ 40 mm/s, weiter bevorzugt ≤ 30 mm/s, weiter bevorzugt ≤ 20 mm/s, weiter bevorzugt ≤ 10 mm/s, noch weiter bevorzugt ≤ 5 mm/s. Ein langsames Herausziehen aus der ersten Lösung bewirkt eine gleichmäßige Beladung der Gefäßendoprothese mit einer Wirkstoffschicht. Darüber hinaus lässt sich über die Geschwindigkeit des Herausziehens auch die Beladung mit Wirkstoff steuern.

Sinnvoll ist es des Weiteren, vor dem Schritt a1) die Gefäßendoprothese von etwaigen Verunreinigungen zu befreien. Dies kann zum einen mechanisch, zum anderen durch entsprechende Lösungsmittel geschehen, wobei insbesondere eine Reinigung mit Wasser, einem Alkohol und/oder einem chlorhaltigen organischen Lösungsmittel sinnvoll ist. Bei dem Alkohol handelt es sich bevorzugt um Ethanol oder Isopropanol, bei dem chlorhaltigen organischen Lösungsmittel bevorzugt um Dichlormethan oder Chloroform. Insbesondere kann die Gefäßendoprothese vor der Benetzung mit dem Wirkstoff durch die erste Lösung in das Lösungsmittel ohne Wirkstoff eingetaucht werden, das auch für die erste Lösung Verwendung findet, um Stoffe, die sich ansonsten in der ersten Lösung ablösen würden, zuvor zu entfernen. Daneben ist auch eine Reinigung mit Hilfe eines mit Lösungsmittel befeuchteten Tuchs o. ä. möglich.

Bei dem verwendeten Wirkstoff handelt es sich insbesondere um ein Arzneimittel bzw. Medikament, das proliferationshemmend wirkt und das gefäßverengende Überwuchern der durch die Gefäßendoprothese offen gehaltenen Stelle verhindert. Insbesondere kann der Wirkstoff ausgewählt sein aus: Tretinoin, Orphanrezeptoragonisten, Elafinderivate, Corticosteroide, Steroidhormone, Paclitaxel, Rapamycin, Sirolimus, Tacrolimus, hydrophobe Proteine sowie zellproliferationsverändernde Substanzen. Es ist auch möglich, Gemische dieser Wirkstoffe zu verwenden. Darüber hinaus können auch Derivate der genannten Wirkstoffe verwendbar sein, wobei unter Derivaten insbesondere Salze, Ester und Amide verstanden werden. Als Steroidhormone können beispielsweise Methylprednisolon, Dexamethason oder Östradiol verwendet werden. Besonders bevorzugt ist die Verwendung von Paclitaxel bzw. Paclitaxelderivaten.

Die Beschichtung der Gefäßendoprothese mit dem Wirkstoff kann auch unter Einsatz von Lösungsvermittlern erfolgen. Als solche sind z. B. bekannt: Phosphatidylcholin, polyethoxyliertes Rizinusöl, Cardiolipin, Cholesterol sowie Gemische hieraus. Bevorzugt ist jedoch der Verzicht auf Lösungsvermittler.

Die oben genannten Verfahrensschritte, insbesondere die Schritte a1) und ggf. a2), können bei Bedarf auch wiederholt werden, d. h. es kann mehrfach ein Wirkstoff, der in einem Lösungsmittel gelöst ist, auf die Gefäßendoprothese aufgebracht und das Innere der Gefäßendoprothese durch Versetzen in eine Rotationsbewegung entfernt werden. Dies führt zu einer erhöhten Beladung mit Wirkstoff. Denkbar ist auch, dass nacheinander unterschiedliche Wirkstoffe aufgebracht werden.

Als weiterer Schritt c) nach dem oben beschriebenen Schritt b) können die mit der ersten Lösung und der Wasser und/oder mindestens einen Alkohol enthaltenden Flüssigkeit benetzten Bereiche der Gefäßendoprothese zumindest teilweise mit einer weiteren Lösung benetzt werden, die ein Polysaccharid enthält. Entsprechend wird die Gefäßendoprothese (vollständig oder partiell) mit einem Polysaccharid beschichtet, wobei sich die Polysaccharidbeschichtung auf der Außenseite des Wirkstoffs befindet, d. h. der Wirkstoff ist weitgehend von einer Polysaccharidschicht abgedeckt. Ggf. kann die Gefäßendoprothese im Inneren noch durch einfaches Abwischen von der Polysaccharidbeschichtung befreit werden, da das Polysaccharid hier keine Funktion ausübt. Unbedingt erforderlich ist eine Entfernung des Polysaccharids jedoch in der Regel nicht, da bereits durch den oben beschriebenen Verfahrensschritt a2) hinreichend dafür Sorge getragen wurde, dass das Innere der Gefäßendoprothese praktisch wirkstofffrei bleibt.

Es hat sich herausgestellt, dass die Polysaccharidbeschichtung ähnlich einem Klebstoff auf der Innenwand des behandelten Gefäßes wirkt, d. h. der Wirkstoff haftet erheblich besser auf der Gefäßwand und wird weniger leicht vom Blutstrom mitgerissen. Vermutlich quillt das Polysaccharid, das im Gegensatz zum Wirkstoff hydrophil ist, in einer wässrigen Umgebung wie Blut leicht auf, wodurch die Übertragung auf die Gefäßinnenwand verbessert wird. Entsprechend kann der Wirkstoff über einen langen Zeitraum seine Wirkung entfalten und aus der Polysaccharidbeschichtung nach und nach in das Gewebe des Gefäßes gelangen. Es konnte gezeigt werden, dass selbst nach ca. 3 Monaten noch signifikante Wirkstoffkonzentrationen nachweisbar sind. Ohne eine Polysaccharidbeschichtung hingegen ist nach 2 bis 3 Tagen kaum noch Wirkstoff im Bereich der Gefäßinnenwand vorhanden, weshalb der durch den Wirkstoff hervorgerufene Schutz vor einer Restenose bei herkömmlichen wirkstoffbeschichteten Stents bereits nach vergleichsweise kurzer Zeit nicht mehr gegeben ist. Für Ballonkatheter ist die Beschichtung der Wirkstoffschicht mit Polysacchariden bereits bekannt, beispielsweise aus der WO 2013/178820 A1.

Ein weiterer Vorteil der Beschichtung mit einem Polysaccharid ist darin zu sehen, dass der Wirkstoff besser an der Gefäßendoprothese fixiert wird. Die Wirkstoffe, wie beispielsweise Paclitaxel, die für eine Beschichtung von Gefäßendoprothesen verwendet werden und proliferationshemmend wirken, sind häufig hoch toxisch, weshalb Ärzte und medizinisches Personal vor Einatmen und Berührung geschützt werden müssen. Durch die Abdeckung des Wirkstoffs mit einer Polysaccharidschicht wird erreicht, dass die Gefäßendoprothese problemlos gehandhabt werden kann, ohne dass sich der Wirkstoff ablöst. Das Risiko, dass Benutzer den Wirkstoff inhalieren oder über die Haut aufnehmen, wird minimiert.

Dadurch, dass zunächst der Wirkstoff und anschließend das Polysaccharid als Polymer jeweils in gelöster Form aufgebracht werden, erreicht man eine gleichmäßige Verteilung des Polysaccharids um die Wirkstoffkristalle der bereits bestehenden Wirkstoffbeschichtung. Darüber hinaus werden Hohlräume zwischen den Wirkstoffpartikeln bzw. zwischen der Oberfläche der Gefäßendoprothese ausgefüllt; die Wirkstoffkristalle werden mit dem Polysaccharid überzogen und ummantelt.

Von besonderem Vorteil ist in diesem Zusammenhang auch, dass die so erhaltene Beschichtung der Gefäßendoprothese mechanisch stabil und flexibel ist, was insofern von Bedeutung ist, als eine Gefäßendoprothese durch einen Katheter häufig durch englumige Blutgefäße an den Bestimmungsort gebracht werden muss. Auch das Verpacken und die Handhabung der Gefäßendoprothese weirden entsprechend sicherer.

Polysaccharide stellen eine hydrophile Beschichtung dar, die in einer wässrigen Umgebung wie Blut eine gewisse Quellung bzw. Aufweichung erfährt. Dies führt dazu, dass der Wirkstoff bei der Aufweitung der Gefäßendoprothese gut auf die Innenwand des Gefäßes übertragen wird. Das erfindungsgemäße Verfahren eignet sich für lipophile Wirkstoffbeschichtungen. Es hat sich nämlich herausgestellt, dass gerade die hydrophilen Polysaccharide gut geeignet sind, dafür zu sorgen, dass lipophile Wirkstoffe während der Aufweitung der Gefäßendoprothese effektiv auf die Innenwände der behandelten Gefäße übertragen werden und eine langanhaltende Wirkstoffkonzentration bewirken. Es wird vermutet, dass, nachdem bei dem erfindungsgemäßen Verfahren zunächst die Wasser und/oder mindestens einen Alkohol enthaltende Flüssigkeit für eine Versprödung der Wirkstoffbeschichtung gesorgt hat, die anschließend aufgebrachten Polysaccharid-Moleküle sich zwischen den Wirkstoffmolekülen ablagern und so für eine homogene Verteilung des Wirkstoffs in der Polysaccharid-Matrix sorgen. Bei durch das erfindungsgemäße Verfahren hergestellten Gefäßendoprothesen wird der Wirkstoff vorteilhafterweise vom im letzten Schritt aufgebrachten Polysaccharid bedeckt.

Das Polysaccharid liegt bevorzugt in einer alkoholischen Lösung vor. Diese kann neben einem oder mehreren Alkoholen insbesondere auch Wasser enthalten. Eine wässrig-alkoholische Lösung ist insofern von Vorteil, als sie das Polysaccharid gut löst, die bereits aufgebrachte Wirkstoffschicht jedoch nicht wieder abträgt. Darüber hinaus sorgt der organische Anteil in der Lösung für eine rasche Trocknung nach der Benetzung. Die Konzentration des Alkohols bzw. der Alkohole in der wässrig-alkoholischen Lösung beträgt typischerweise 10 bis 70 % (v/v), bevorzugt 30 bis 65 % (v/v), weiter bevorzugt 50 bis 60 % (v/v) und besonders bevorzugt ca. 55 % (v/v). Als Alkohol verwendbar sind solche Alkohole, die das Polysaccharid lösen. In der Regel sind derartige Alkohole auch mit Wasser mischbar. Bevorzugt sind Ethanol, Methanol und Isopropanol, besonders bevorzugt ist Ethanol. Im Anschluss an und/oder bereits während der Benetzung mit der ein Polysaccharid enthaltenden Lösung kann wiederum die Gefäßendprothese in Rotation versetzt werden bzw. sich in Rotation befinden, um überschüssige Lösung zu entfernen und eine schnellere Trocknung zu bewirken.

Ggf. können auch die Schritte b) und c) vereinigt werden, weil die wäßrigealkoholische Lösung, in der das Polysaccharid gelöst ist, ebenfalls geeignet ist, die erwünschte Versprödung der Wirkstoffoberfläche herbeizuführen. In diesem Fall dient Schritt b/c) einem zweifachen Zweck, zum einen wird die Wirkstoffschicht poröser, zum anderen wird die Wirkstoffschicht mit Polysaccharid abgedeckt, wobei das Polysaccharid auch in die einzelnen Hohl- und Zwischenräume eindringt, die sich in der Wirkstoffschicht ausbilden.

Die mittlere Molmasse des Polysaccharids beträgt zweckmäßigerweise 10.000 bis 100.000.000 Da. Als besonders zweckmäßig hat sich eine mittlere Molmasse zwischen 20.000 und 80.000 Da herausgestellt. Bevorzugt sind verzweigte Polysaccharide. Der Polysaccharid-Gehalt der weiteren Lösung beträgt vorzugsweise 1 bis 15 Gew.-%, weiter bevorzugt 2 bis 10 Gew.-% und besonders bevorzugt 3 bis 8 Gew.-%.

Bei dem Polysaccharid handelt es sich bevorzugt um ein verzweigtes Polysaccharid. Geeignet sind auch Gemische aus mehreren Polysacchariden und modifizierte Polysaccharide. Bevorzugt sind Dextrane, insbesondere natürliche Dextrane. Bei Dextranen handelt es sich um hochmolekulare, verzweigte Polymere, die sich aus Glucoseeinheiten zusammensetzen. Sie werden u. a. von Bakterien der Gattung *Leuconostoc* hergestellt. Verwendung finden sie als Blutplasma-Ersatzmittel oder als Träger in der Chromatographie. Dextrane wirken zudem antithrombogen.

Bei dem Dextran kann es sich insbesondere um ein natürliches Dextran handeln. Besonders bevorzugt ist Dextran 40 mit einer mittleren Molmasse von ca. 40.000 Da.

Neben Dextranen können jedoch grundsätzlich auch andere Polysaccharide Verwendung finden. Ein Beispiel für ein verwendbares modifiziertes Polysaccharid ist Hydroxyethylstärke (HES).

Sämtliche Benetzungen der Oberfläche der Gefäßendoprothese mit einer Flüssigkeit (erste Lösung, Wasser und/oder mindestens einen Alkohol enthaltende Flüssigkeit oder ein Polysaccharid enthaltende weitere Lösung) können durch Eintauchen in die Flüssigkeit erfolgen. Das Eintauchen dauert dabei in der Regel max. 1 min, typischerweise 10 bis 30 s, wobei für das Eintauchen in die Wasser/Alkohol enthaltende Flüssigkeit eine Zeit von mindestens 50 s bevorzugt ist. Alternativ zur Benetzung durch Eintauchen kann die Benetzung auch auf andere Weise erfolgen, bspw. durch Besprühen oder Auftropfen. Nach den einzelnen Benetzungsschritten lässt man die Oberfläche der Gefäßendoprothese zunächst trocknen, bevor weitere Verfahrensschritte durchgeführt werden. Das Trocknen kann durch Einwirkenlassen eines Luft- oder Gasstroms unterstützt werden. Auch das Versetzen der Gefäßendoprothese in Rotation unterstützt die Trocknung.

Bei Bedarf kann die Oberfläche der Gefäßendoprothese vor dem Aufbringen des Wirkstoffs durch mechanische, chemische oder thermische Einwirkung vergrößert, beispielsweise aufgeraut oder angeätzt werden. Auf diese Weise erhält die Oberfläche eine gröbere Struktur, so dass die Wirkstoffbeladung erhöht werden kann. Die so erzeugten Vertiefungen in der Oberfläche können bspw. eine Tiefe und einen Durchmesser von 5 - 50 µm aufweisen.

Sämtliche Verfahrensschritte können bei Raumtemperatur durchgeführt werden.

Neben dem beschriebenen erfindungsgemäßen Verfahren betrifft die Erfindung auch eine Gefäßendoprothese, deren Außenseite zumindest teilweise eine Beschichtung mit einem hydrophoben Wirkstoff aufweist und die durch das oben beschriebene Verfahren erhältlich ist. Die Beschichtung mit dem Wirkstoff sowie auch dem Polysaccharid kann dabei die gesamte äußere Oberfläche der Gefäßendoprothese betreffen oder lediglich Teilbereiche. Von Bedeutung ist, dass die Wirkstoffschicht durch Schritt b) versprödet wird, um einen höheren Abrieb und eine bessere Übertragung des Wirkstoffs auf die Gefäßwand zu bewirken. Zusätzlich ist es sinnvoll, das Innere der Gefäßendoprothese so weit wie möglich wirkstofffrei zu halten, indem die Gefäßendoprothese nach Beschichtung mit dem Wirkstoff in Rotation versetzt wird, um dort Endothelwachstum zu ermöglichen, das für die Einbettung der Gefäßendoprothese in das Gefäß sorgt. Auf diese Weise wird die Gefäßendoprothese schnell in das Körpergewebe integriert, wobei gleichzeitig der auf der äußeren Oberfläche der Gefäßendoprothese vorhandene Wirkstoff Restenosen durch unkontrolliertes Zellwachstum wirkungsvoll verhindert. Bei der erfindungsgemäßen Gefäßendoprothese kann es sich z. B. um einen Stent handeln, wie er im Übrigen zum Offenhalten eines Gefäßlumens bekannt ist. Ein solcher Stent weist typischerweise eine Röhrenform auf und besteht aus einer Geflecht- oder Gitterstruktur aus Metall oder Kunststoff. Der Stent kann eine komprimierte Form einnehmen, um ihn durch einen Katheter an den Zielort einführen zu können. Am Zielort wird der Stent dann aufgeweitet, so dass er seine expandierte Form einnimmt.

Besonders vorteilhaft ist es, wenn der Wirkstoff darüber hinaus von einem Polysaccharid, insbesondere einem Dextran bedeckt wird. Dies gilt unabhängig davon, ob zuvor die weiteren beschriebenen Schritte a2) und a3) durchgeführt wurden. Das Polysaccharid bewirkt eine gute Anhaftung des Wirkstoffs an der Gefäßinnenwand, so dass eine Mitnahme des Wirkstoffs durch den Blutstrom weitgehend verhindert wird. Entsprechend können an der Gefäßinnenwand selbst nach Monaten noch erhebliche Wirkstoffkonzentrationen nachgewiesen werden, während bei herkömmlichen wirkstoffbeschichteten Gefäßendoprothesen teilweise bereits nach wenigen Tagen kaum noch Wirkstoff an der Gefäßinnenwand vorhanden ist.

## Patentansprüche

1. Verfahren zur Beschichtung einer Gefäßendoprothese mit folgenden Schritten
a1) zumindest teilweise Benetzung der Gefäßendoprothese mit einer ersten Lösung eines hydrophoben Wirkstoffs
b) zumindest teilweise Benetzung der mit der ersten Lösung des Wirkstoffs benetzten Bereiche der Gefäßendoprothese mit einer Wasser und/oder mindestens einen Alkohol enthaltenden Flüssigkeit,
wobei vor der Benetzung der Gefäßendoprothese mit der Wasser und/oder mindestens einen Alkohol enthaltenden Flüssigkeit eine Trocknung der Gefäßendoprothese und eine Auskristallisation des Wirkstoffs erfolgt und wobei die Wasser und/oder mindestens einen Alkohol enthaltende Flüssigkeit einen Alkohol und/oder ein Keton enthält.

2. Verfahren nach Anspruch 1, **dadurch gekennzeichnet, dass** die Konzentration des Alkohols und/oder Ketons in der Flüssigkeit 10 bis 70 % (v/v), bevorzugt 20 bis 40 % (v/v) beträgt.

3. Verfahren nach Anspruch 1 oder 2, **dadurch gekennzeichnet, dass** die Flüssigkeit Ethanol, Methanol, Aceton und/oder Isopropanol enthält.

4. Verfahren nach einem der Ansprüche 1 bis 3, **dadurch gekennzeichnet, dass** die Gefäßendoprothese nach der Benetzung mit der Wasser und/oder mindestens einen Alkohol enthaltenden Flüssigkeit über einen Zeitraum ≥ 2 min, bevorzugt ≥ 3 min, besonders bevorzugt ≥ 5 min getrocknet wird.

5. Verfahren nach einem der Ansprüche 1 bis 4, umfassend als weiteren Schritt:
a2) Versetzen der Gefäßendoprothese in eine Rotationsbewegung um die Längsachse der Gefäßendoprothese,
wobei Schritt a2) nach Schritt a1) und vor Schritt b) durchgeführt wird.

6. Verfahren nach Anspruch 5, **dadurch gekennzeichnet, dass** die Gefäßendoprothese in eine Rotationsbewegung um die Längsachse mit einer Umdrehungsgeschwindigkeit von mindestens 1.000 U/min, bevorzugt mindestens 2.000 U/min, besonders bevorzugt mindestens 5.000 U/min versetzt wird.

7. Verfahren nach einem der Ansprüche 1 bis 6, umfassend als weiteren Schritt:
a3) Ausübung einer radial wirkenden mechanischen Kraft auf die Außenseite der Gefäßendoprothese,
wobei Schritt a3) nach Schritt a1) und vor Schritt b) oder nach Schritt a2) und vor Schritt b) durchgeführt wird.

8. Verfahren nach Anspruch 7, **dadurch gekennzeichnet, dass** die radial wirkende mechanische Kraft dadurch auf die Außenseite der Gefäßendoprothese ausgeübt wird, dass die Gefäßendoprothese unter Ausübung eines Drucks über eine Oberfläche gerollt wird.

9. Verfahren nach einem der Ansprüche 1 bis 8, **dadurch gekennzeichnet, dass** die erste Lösung als Lösungsmittel Chloroform oder Dichlormethan enthält.

10. Verfahren nach einem der Ansprüche 1 bis 9, **dadurch gekennzeichnet, dass** die Gefäßendoprothese aus der ersten Lösung mit einer Geschwindigkeit von ≤ 50 mm/s, weiter bevorzugt ≤ 40 mm/s, weiter bevorzugt ≤ 30 mm/s, weiter bevorzugt ≤ 20 mm/s, weiter bevorzugt ≤ 10 mm/s, noch weiter bevorzugt ≤ 5 mm/s herausbewegt wird.

11. Verfahren nach einem der Ansprüche 1 bis 10, umfassend als weiteren Schritt:
c) zumindest teilweise Benetzung der mit der ersten Lösung und mit der Wasser und/oder mindestens einen Alkohol enthaltenden Flüssigkeit benetzten Bereiche der Gefäßendoprothese mit einer weiteren Lösung, die ein Polysaccharid enthält,
wobei Schritt c) nach Schritt b) durchgeführt wird.

12. Verfahren nach Anspruch 11, **dadurch gekennzeichnet, dass** die mittlere Molmasse des Polysaccharids 10.000 bis 100.000.000 Da, bevorzugt 20.000 bis 80.000 Da beträgt.

13. Verfahren nach Anspruch 11 oder 12, **dadurch gekennzeichnet, dass** das Polysaccharid ein Dextran ist.

14. Gefäßendoprothese, deren Außenseite zumindest teilweise eine Beschichtung mit einem hydrophoben Wirkstoff aufweist, erhältlich durch ein Verfahren nach einem der Ansprüche 1 bis 13.

15. Gefäßendoprothese nach Anspruch 14, erhältlich durch ein Verfahren nach einem der Ansprüche 11 bis 13, **dadurch gekennzeichnet, dass** der Wirkstoff zumindest teilweise von dem Polysaccharid bedeckt wird.

## Claims

1. A method for coating a vascular endoprosthesis, comprising the following steps:
a1) at least partial wetting of the vascular endoprosthesis with a first solution of a hydrophobic active ingredient, and
b) at least partial wetting of the areas of the vascular endoprosthesis wetted with the solution of the active ingredient with a liquid comprising water and/or at least one alcohol,
wherein before the wetting of the vascular endoprosthesis with the liquid comprising water and/or at least one alcohol a drying of the vascular endoprosthesis and a crystallization of the active ingredient takes place, wherein the liquid comprising water and/or at least one alcohol comprises an alcohol and/or a ketone.

2. The method as claimed in claim 1, **characterized in that** the concentration of the alcohol and/or ketone in the liquid is 10 to 70% (v/v), and preferably 20 to 40% (v/v).

3. The method as claimed in claim 1 or 2, **characterized in that** the liquid comprises ethanol, methanol, acetone, and/or isopropanol.

4. The method as claimed in any of claims 1 to 3, **characterized in that** the vascular endoprosthesis, after wetting with the liquid comprising water and/or at least one alcohol, is dried for a period of ≥ 2 min, preferably ≥ 3 min, and particularly preferably ≥ 5 min.

5. The method as claimed in any of claims 1 to 4, comprising as a further step:
a2) causing the vascular endoprosthesis to rotate about the longitudinal axis of the vascular endoprosthesis,
wherein step a2) is carried out after step a1) and before step b).

6. The method as claimed in claim 5, **characterized in that** the vascular endoprosthesis is caused to rotate about the longitudinal axis at a rotational speed of at least 1,000 rpm, preferably at least 2,000 rpm, and particularly preferably at least 5,000 rpm.

7. The method as claimed in any of claims 1 to 6, comprising as a further step:
a3) Exerting a radially acting mechanical force on the outer side of the vascular endoprosthesis,
wherein step a3) is carried out after step a1) and before step b) or after step a2) and before step b).

8. The method as claimed in claim 7, **characterized in that** the radially acting mechanical force is exerted on the outside of the vascular endoprosthesis such that the vascular endoprosthesis is rolled over a surface while exerting pressure.

9. The method as claimed in any of claims 1 to 8, **characterized in that** the first solution comprises chloroform or dichloromethane as a solvent.

10. The method as claimed in any of claims 1 to 9, **characterized in that** the vascular endoprosthesis is removed from the first solution at a rate of ≤ 50 mm/sec, more preferably ≤ 40 mm/sec, more preferably ≤ 30 mm/sec, more preferably ≤ 20 mm/sec, more preferably ≤ 10 mm/sec, and even more preferably ≤ 5 mm/sec.

11. The method as claimed in any of claims 1 to 10, comprising as a further step:
c) at least partial wetting of the areas of the vascular endoprosthesis wetted with the first solution and the liquid comprising water and/or at least one alcohol with a further solution comprising a polysaccharide,
wherein step c) is carried out after step b).

12. The method as claimed in claim 11, **characterized in that** the average molecular weight of the polysaccharide is 10,000 to 100,000,000 Da, and preferably 20,000 to 80,000 Da.

13. The method as claimed in claim 11 or 12, **characterized in that** the polysaccharide is a dextran.

14. A vascular endoprosthesis, the outer side of which is at least partially coated with a hydrophobic active ingredient, obtainable by a method as claimed in any of claims 1 to 13.

15. The vascular endoprosthesis as claimed in claim 14, obtainable by a method as claimed in any of claims 11 to 13, **characterized in that** the active ingredient is at least partially coated with the polysaccharide.

## Revendications

1. Procédé de revêtement d'une endoprothèse vasculaire avec des étapes suivantes :
a1) d'humidification au moins partielle de l'endoprothèse vasculaire avec une première solution d'un principe actif hydrophobe,
b) d'humidification au moins partielle des zones, humidifiée avec la première solution du principe actif, de l'endoprothèse vasculaire avec une eau et/ou du liquide contenant au moins un alcool,
dans lequel avant l'humidification de l'endoprothèse vasculaire avec l'eau et/ou du liquide contenant au moins un alcool, un séchage de l'endoprothèse vasculaire et une cristallisation du principe actif sont effectués et dans lequel l'eau et/ou du liquide contenant au moins un alcool contiennent un alcool et/ou une cétone.

2. Procédé selon la revendication 1, **caractérisé en ce que** la concentration de l'alcool et/ou de la cétone dans le liquide est de 10 à 70 % (v/v), de manière préférée de 20 à 40 % (v/v).

3. Procédé selon la revendication 1 ou 2, **caractérisé en ce que** le liquide contient de l'éthanol, du méthanol, de l'acétone et/ou de l'isopropanol.

4. Procédé selon l'une quelconque des revendications 1 à 3, **caractérisé en ce que** l'endoprothèse vasculaire est séchée après l'humidification avec l'eau et/ou du liquide contenant au moins un alcool pendant une période ≥ 2 min, de manière préférée ≥ 3 min, de manière particulièrement préférée ≥ 5 min.

5. Procédé selon l'une quelconque des revendications 1 à 4, comprenant en tant qu'étape supplémentaire :
a2) la mise de l'endoprothèse vasculaire en un mouvement de rotation autour de l'axe longitudinal de l'endoprothèse vasculaire,
dans lequel l'étape a2) est mise en oeuvre après l'étape a1) et avant l'étape b).

6. Procédé selon la revendication 5, **caractérisé en ce que** l'endoprothèse vasculaire est mise en un mouvement de rotation autour de l'axe longitudinal à une vitesse de tour complet d'au moins 1.000 T/min, de manière préférée d'au moins 2.000 T/min, de manière particulièrement préférée d'au moins 5.000 T/min.

7. Procédé selon l'une quelconque des revendications 1 à 6, comprenant en tant qu'étape supplémentaire :
a3) l'application d'une force mécanique agissant radialement sur le côté extérieur de l'endoprothèse vasculaire,
dans lequel l'étape a3) est mise en oeuvre après l'étape a1) et avant l'étape b) ou après l'étape a2) et avant l'étape b).

8. Procédé selon la revendication 7, **caractérisé en ce que** la force mécanique agissant radialement est appliquée sur le côté extérieur de l'endoprothèse vasculaire **en ce que** l'endoprothèse vasculaire est roulée sur une surface en appliquant une pression.

9. Procédé selon l'une quelconque des revendications 1 à 8, **caractérisé en ce que** la première solution contient en tant que solvant du chloroforme ou du chlorure de méthylène.

10. Procédé selon l'une quelconque des revendications 1 à 9, **caractérisé en ce que** l'endoprothèse vasculaire est retirée de la première solution à une vitesse ≤ 50 mm/s, de manière davantage préférée ≤ 40 mm/s, de manière davantage préférée ≤ 30 mm/s, de manière davantage préférée ≤ 20 mm/s, de manière davantage préférée ≤ 10 mm/s, de manière encore plus préférée ≤ 5 mm/s.

11. Procédé selon l'une quelconque des revendications 1 à 10, comprenant en tant qu'étape supplémentaire :
c) l'humidification au moins partielle des zones, humidifiés avec la première solution et avec l'eau et/ou du liquide contenant au moins un alcool, de l'endoprothèse vasculaire avec une autre solution, qui contient un polysaccharide,
dans lequel l'étape c) est mise en oeuvre après l'étape b).

12. Procédé selon la revendication 11, **caractérisé en ce que** la masse molaire moyenne du polysaccharide est de 10.000 à 100.000.000 Da, de manière préférée de 20.000 à 80.000 Da.

13. Procédé selon la revendication 11 ou 12, **caractérisé en ce que** le polysaccharide est un dextrane.

14. Endoprothèse vasculaire, dont le côté extérieur présente au moins en partie un revêtement avec un principe actif hydrophobe, pouvant être obtenu par un procédé selon l'une quelconque des revendications 1 à 13.

15. Endoprothèse vasculaire selon la revendication 14, pouvant être obtenue par un procédé selon l'une quelconque des revendications 11 à 13, **caractérisée en ce que** le principe actif est recouvert au moins en partie du polysaccharide.
